(19) Europäisches Patentamt
European Patent Office
Office européen des brevets

(11) **EP 2 898 318 B1**

(12) **FASCICULE DE BREVET EUROPEEN**

(45) Date de publication et mention de la délivrance du brevet:
**22.07.2020 Bulletin 2020/30**

(21) Numéro de dépôt: **13762520.8**

(22) Date de dépôt: **17.09.2013**

(51) Int Cl.:
*G01N 27/18* (2006.01)          *G01N 30/66* (2006.01)
*G01L 21/12* (2006.01)          *G01N 33/00* (2006.01)

(86) Numéro de dépôt international:
**PCT/EP2013/069239**

(87) Numéro de publication internationale:
**WO 2014/044663 (27.03.2014 Gazette 2014/13)**

(54) **CAPTEUR DE FLUX THERMIQUE ET CAPTEUR DE GAZ COMPORTANT AU MOINS UN TEL CAPTEUR**

WÄRMEFLUSSSENSOR UND GAS SENSOR MIT MINDESTENS EINEM SOLCHEN SENSOR

THERMAL FLOW SENSOR AND GAS SENSOR COMPRISING AT LEAST ONE SUCH SENSOR

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **19.09.2012 FR 1258794**

(43) Date de publication de la demande:
**29.07.2015 Bulletin 2015/31**

(73) Titulaire: **Commissariat à l'Énergie Atomique et aux Énergies Alternatives 75015 Paris (FR)**

(72) Inventeurs:
 • **RUELLAN, Jérémie 38000 Grenoble (FR)**
 • **DURAFFOURG, Laurent 38500 Voiron (FR)**
 • **ARCAMONE, Julien 38000 Grenoble (FR)**
 • **ANDREUCCI, Philippe 38430 Moirans (FR)**
 • **COLINET, Eric 38000 Grenoble (FR)**

(74) Mandataire: **Brevalex 95, rue d'Amsterdam 75378 Paris Cedex 8 (FR)**

(56) Documents cités:

US-A- 5 379 630          US-A1- 2012 024 043
US-B1- 6 169 965

• WEI LI ET AL: "Single-walled carbon nanotube pirani vacuum gauge", SOLID-STATE AND INTEGRATED CIRCUIT TECHNOLOGY (ICSICT), 2010 10TH IEEE INTERNATIONAL CONFERENCE ON, IEEE, PISCATAWAY, NJ, USA, 1 novembre 2010 (2010-11-01), pages 1407-1409, XP031835238, ISBN: 978-1-4244-5797-7
• TAKESHI KAWANO ET AL: "An Electrothermal Carbon Nanotube Gas Sensor", NANO LETTERS, vol. 7, no. 12, 1 décembre 2007 (2007-12-01), pages 3686-3690, XP055047103, ISSN: 1530-6984, DOI: 10.1021/nl071964s
• MASANORI KUBOTA ET AL: "Silicon sub-micron-gap deep trench Pirani vacuum gauge for operation at atmospheric pressure;Silicon sub-micron-gap deep trench Pirani vacuum gauge for operation at atmospheric pressure", JOURNAL OF MICROMECHANICS & MICROENGINEERING, INSTITUTE OF PHYSICS PUBLISHING, BRISTOL, GB, vol. 21, no. 4, 045034, 24 mars 2011 (2011-03-24), pages 1-7, XP020188927, ISSN: 0960-1317, DOI: 10.1088/0960-1317/21/4/045034
• BEDO G ET AL: "Comparison of different micromechanical vacuum sensors", SENSORS AND ACTUATORS A: PHYS, ELSEVIER BV, NL, vol. 85, no. 1-3, 25 August 2000 (2000-08-25), pages 181-188, XP004214468, ISSN: 0924-4247, DOI: 10.1016/S0924-4247(00)00383-6

**Description**

## DOMAINE TECHNIQUE ET ART ANTÉRIEUR

La présente invention se rapporte à un capteur de flux thermique, qui peut être destiné à la mesure de la concentration d'un gaz ou capteur TCD ou à la mesure de très faibles pressions formant ainsi une jauge Pirani.

De façon générale, on entend par capteur de flux thermique, tout capteur mesurant un échange de chaleur entre le corps du capteur (membrane) et le milieu fluidique dans lequel le capteur est disposé.

Un capteur de flux thermique est placé dans un environnement contenant l'élément à analyser, un analyte dans un gaz porteur dans le cas d'un capteur de gaz ou simplement un certain nombre de molécules de gaz dans le cas d'une jauge Pirani.

Le document WO2001/044547 décrit un capteur TCD ("Thermal Conductivity Detectors" utilisant la variation de la conductivité thermique pour déterminer la composition de l'environnement gazeux dans lequel il est disposé. Celui-ci est disposé en sortie d'une colonne de chromatographie. Ce capteur TCD comporte une plaque support allongée, un élément chauffant situé sur la plaque support. La variation de tension est mesurée aux bornes de la plaque support chauffée pour déterminer la variation de résistance électrique qui dépend de la température de la plaque support et qui est représentative des échanges thermiques entre la plaque support et l'environnement gazeux; ces échanges thermiques dépendent de la composition de l'environnement gazeux. Un tel capteur est de fabrication complexe puisqu'elle nécessite la réalisation de la plaque support et ensuite celle de l'élément chauffant.

Une jauge Pirani est formée d'un filament suspendu de taille micrométrique, celui-ci est placé dans une enceinte dont on veut mesurer la pression et est chauffé. La mesure de la pression est déduite de la quantité de chaleur perdue par le filament par suite de la conduction gazeuse : les molécules qui rencontrent le filament absorbent une partie de son énergie. Un exemple de jauge Pirani employant des nanotubes de carbone suspendus est décrit dans le document: Wei et al. "Single-walled carbon nanotube pirani vacuum gauge" dans Proceedings of the 2010 10th Solid State and Integrated Circuit Technology (ICSICT) Conférence, 1 novembre 2010, pages 1407-1409.

Ces capteurs présentent l'inconvénient de ne pas permettre de s'affranchir complètement des phénomènes survenant à l'extérieur du capteur lui-même, par exemple des dérives en température.

En outre, de manière générale on cherche à réduire la taille des capteurs pour faciliter leur intégration et accroître le nombre de domaines d'application. Cependant une telle miniaturisation provoque une augmentation du Bruit de Flicker, également désigné "Bruit en 1/f".

## EXPOSÉ DE L'INVENTION

C'est par conséquent un but de la présente invention d'offrir un capteur de flux thermique permettant de s'affranchir au moins en partie des phénomènes survenant à l'extérieur du capteur et de taille réduite.

C'est également un but de la présente invention d'offrir un capteur de flux thermique de réalisation plus simple que celle des capteurs de l'état de la technique.

Le but précédemment énoncé est atteint par un dispositif de mesure de flux thermique comportant au moins un nanofil suspendu destiné à être disposé dans l'environnement à analyser, des moyens de chauffage dynamique dudit nanofil et des moyens des mesure de la variation dynamique de la tension électrique du nanofil.

On entend par "chauffage dynamique" l'application d'un courant électrique alternatif de période donnée et par "variation dynamique de la tension électrique" la variation de tension due à la variation de la résistance électrique du nanofil qui résulte de la variation dans le temps de la température du nanofil.

La mise en œuvre d'un ou plusieurs nanofils permet d'avoir des élément sensibles présentant une constante de temps thermique relativement faible, ce qui permet d'avoir une variation de température du dispositif qui suit la variation de courant et donc de pouvoir appliquer un signal d'excitation dynamique.

L'application de signaux d'excitation dynamique permet de rendre le capteur insensible aux dérives lentes, par exemple les dérives de la température du milieu.

Le dispositif de mesure selon l'invention présente une résolution élevée de la mesure de la température du système.

En outre, grâce à l'invention, le signal d'excitation peut présenter une fréquence suffisante pour réduire le bruit de Flicker.

Dans un mode de réalisation particulièrement, avantageux, le dispositif de mesure comporte au moins deux nanofils ou éléments suspendus.

Dans un mode de réalisation, des moyens de chauffage dynamique dudit nanofil et des moyens des mesure de la variation dynamique de la tension électrique du nanofil sont prévus.

Dans un autre mode de réalisation, l'un des nanofils sert au chauffage par effet Joule, et l'autre servant de mesure. Les parties « excitation » et « détection » sont alors découplées permettant une moins grande sensibilité par rapport à la dérive des paramètres extérieurs, tels que la température.

En multipliant le nombre de nanofils, le capteur disposé dans un canal dans lequel circule un gaz peut permettre de caractériser des transferts de chaleur dans le canal.

Cette invention permet en outre de réaliser des détecteurs performants de très petites tailles co-intégrables avec des technologies de la microélectronique et compatibles CMOS (VLSI).

Un tel capteur est particulièrement intéressant en tant que capteur de gaz utilisé dans et/ou en sortie d'une micro-colonne de chromatographie en phase gazeuse.

La présente invention a alors pour objet un capteur de flux thermique comportant au moins un premier élément suspendu par rapport à un support, ledit premier élément suspendu étant en un matériau conducteur électrique, des premiers moyens de polarisation dudit élément suspendu et des premiers moyens de mesure de la variation de la tension électrique aux bornes de l'élément suspendu, ledit premier élément suspendu étant formé par au moins un nanofil et lesdits premiers moyens de polarisation sont formés pas une source de courant alternatif dont l'intensité assure un échauffement du premier élément suspendu par effet Joule. Le premier élément suspendu assure à la fois l'excitation thermique et la détection de la variation de résistance induite par l'échauffement alternatif.

Dans un exemple de réalisation, le capteur comporte un deuxième élément suspendu, formé par au moins un nanofil, sensiblement parallèle au premier élément suspendu et distant du premier élément suspendu d'une distance donnée, des deuxièmes moyens de polarisation dudit deuxième élément suspendu et des deuxièmes moyens de mesure de la variation de la tension électrique aux bornes du deuxième élément suspendu, les deuxièmes moyens de polarisation étant formés par une source de courant alternatif de fréquence différente de celle des premiers moyens de polarisation ou par une source de courant constant. Le deuxième élément suspendu assure alors uniquement la détection de la variation de résistance induite par l'échauffement alternatif du premier élément suspendu.

Dans un autre exemple de réalisation, le capteur comporte n premiers éléments suspendus disposés sensiblement parallèlement entre eux, n étant un entier positif strictement supérieur à 1, chaque premier élément suspendu comportant des premiers moyens de polarisation dudit premier élément suspendu et des premiers moyens de mesure de la variation de la tension électrique aux bornes du premier élément suspendu, lesdits premiers moyens de polarisation des n premiers éléments étant formés respectivement par une source de courant alternatif de même fréquence ou de fréquence différente dont l'intensité assure un échauffement du premier élément suspendu correspondant par effet Joule. Les n éléments suspendus du premier type assurent à la fois l'excitation thermique et la détection de la variation de résistance induite par l'échauffement alternatif.

Selon la présente invention, le capteur comporte m deuxième éléments suspendus, sensiblement parallèles entre eux, m étant un entier positif strictement supérieur à 1, chaque deuxième élément suspendu comportant des deuxièmes moyens de polarisation dudit deuxième élément suspendu et des deuxièmes moyens de mesure de la variation de la tension électrique aux bornes du deuxième élément suspendu, les deuxièmes moyens de polarisation étant formés par une source de courant alternatif de fréquence différente de celle des premiers moyens de polarisation ou par une source de courant constant. Les entiers n et m peuvent être égaux ou différents suivant les modes de réalisation.

De manière avantageuse, les premiers moyens de polarisation des n premiers éléments suspendus, respectivement les deuxièmes moyens de polarisation des m seconds éléments suspendus sont communs.

Par exemple, le premier élément suspendu peut être encadré entre m deuxièmes éléments suspendus et/ou le deuxième élément suspendu peut être encadré entre n premiers éléments suspendus.

Dans un exemple de réalisation, les n premiers éléments suspendus et les m deuxièmes éléments suspendus sont répartis dans un plan ou plusieurs plans parallèles, dans ce derniers cas le capteur s'étend alors les trois dimensions. Ce type de capteur peut permettre de caractériser des transferts de chaleur dans un canal fluidique.

Dans un autre exemple de réalisation où le capteur comporte un premier élément suspendu, celui-ci peut avantageusement comporter au moins deux masses thermiques reliées au support, les masses thermiques étant disposées de part et d'autre du premier élément suspendu. Dans le cas où le capteur comporte n premiers éléments suspendus, le capteur peut avantageusement comprendre et n+1 masses thermiques, lesdites n + 1 masses thermiques étant disposées de sorte que chaque premier élément est encadré par deux masses. Les masses formant des masses thermostatées améliorent les échanges thermiques.

De manière avantageuse, le ou les premiers ou seconds éléments suspendus ont respectivement une largeur comprise entre 10nm et 1$\mu$m, une longueur comprise entre 1$\mu$m et 100$\mu$m, une épaisseur comprise entre 10nm et 1$\mu$m, la distance séparant les premiers éléments suspendus des seconds éléments suspendus est comprise entre 10 nm et 10 $\mu$m.

Par exemple, un capteur de flux thermique selon l'invention comportant un premier élément suspendu encadré par m deuxièmes éléments suspendus, peut être destiné être disposé dans un canal. Lesdits éléments suspendus peuvent alors être disposés parallèlement à l'axe du canal de sorte à déterminer les échanges de chaleur transversaux dans le gaz à l'intérieur du canal.

La présente invention a également pour objet un système de détermination de la concentration d'un environnement gazeux comportant au moins un capteur de flux thermique selon l'invention, une électronique de traitement des valeurs de tension électrique délivrées par le capteur de flux thermique.

La présente invention a également pour objet un système de détermination de la pression dans un environnement gazeux comportant au moins un capteur de flux thermique selon l'invention, une électronique de traitement des valeurs de tension électrique délivrées par le capteur de flux thermique.

La présente invention a également pour objet un dispositif d'analyse d'un gaz ou mélange de gaz comportant une colonne de chromatographie en phase gazeuse et au moins un système de détermination de la concentration selon l'invention, ledit système de détermination étant disposé dans un canal connecté à la sortie de la colonne de chromatographie en phase gazeuse.

## BRÈVE DESCRIPTION DES DESSINS

La présente invention sera mieux comprise à l'aide de la description qui va suivre et des dessins en annexes sur lesquels :

- la figure 1 est une représentation schématique d'un exemple de capteur à un nanofil,
- la figure 2 est une représentation du modèle thermique du capteur de la figure 1,
- la figure 3 est une représentation schématique d'un exemple de capteur à deux nanofils,
- la figure 4 est une représentation du schéma électrique équivalent du capteur de la figure 3,
- la figure 5 est une représentation schématique d'un exemple de capteur à trois nanofils,
- la figure 6 est une représentation schématique d'un exemple de capteur à n nanofils,
- la figure 7A est une représentation graphique schématique de la température de différentes nanofils d'un capteur selon l'invention en fonction de leur position dans un canal,
- la figure 7B est une représentation en coupe transversale d'un canal muni d'un capteur à plusieurs nanofils selon l'invention,
- les figures 8A et 8B sont des représentations schématiques de capteurs présentant plusieurs nanofils répartis en plusieurs couches,
- les figures 9A et 9B sont des vues de dessus et de côté respectivement d'un autre exemple d'un dispositif de mesure mettant en œuvre des masses thermostatées,
- les figures 9C et 9D sont des vues de dessus d'autres exemples de réalisation d'un dispositif de mesure mettant en œuvre des masses thermostatées.
- les figures 10A à 10H sont des représentations schématiques de différentes étapes d'un exemple de procédé de réalisation d'un capteur selon l'invention.

## EXPOSÉ DÉTAILLÉ DE MODES DE RÉALISATION PARTICULIERS

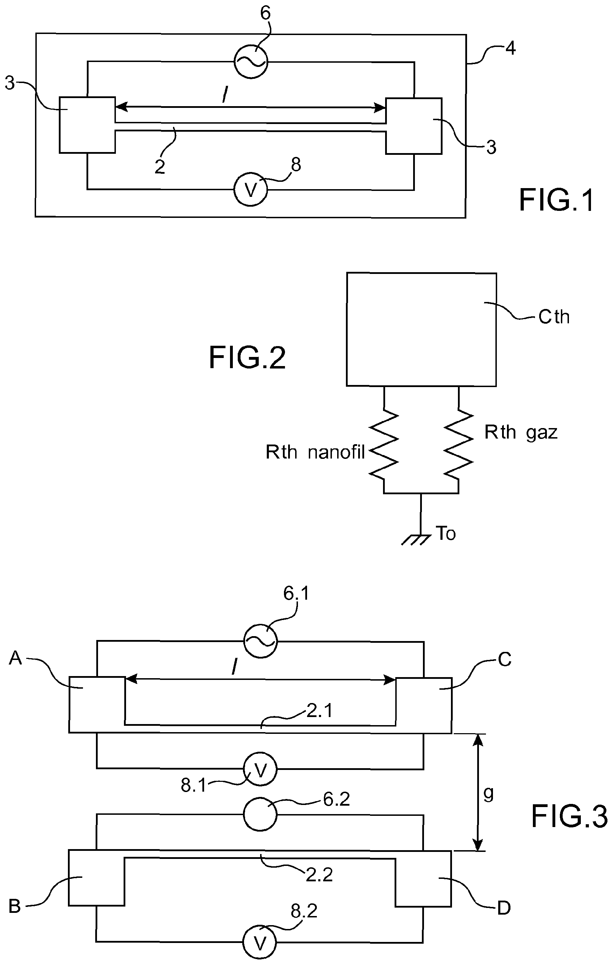

Sur la figure 1, on peut voir un exemple de dispositif de mesure selon l'invention comportant un élément suspendu 2 par rapport à un support 4 au niveau de ses deux extrémités longitudinales. Le nanofil 2 est ancré sur le support par des plots d'ancrage 3 formant plots de contact. L'élément suspendu 2 est un nanofil.

Dans la description qui va suivre, il est fait usage de l'expression "les bornes du nanofil", il s'agit des plots de contact du nanofil sur le support qui sont en général formés sur les plots d'ancrage du nanofil sur le support.

L'élément 2 est en un matériau conducteur électrique, par exemple en matériau semi-conducteur dopé ou non, par exemple en silicium, dopé N ou P, en TiN, en métal ou alliages métalliques ou encore en siliciures, par exemple en NiSi).

La longueur du nanofil est avantageusement comprise entre 1 $\mu$m et 100 $\mu$m et la largeur et l'épaisseur du nanofil sont avantageusement comprises entre 10 nm et 1 $\mu$m.

Le dispositif de mesure comporte également des moyens d'excitation dynamique ou modulée formés par une source de courant alternatif 6 reliée au nanofil 2 et des moyens de détection de l'amplitude de variation de tension aux bornes du nanofil, formés par un voltmètre 8.

La source de courant alternatif 6 forme des moyens de chauffage dynamique par effet Joule du nanofil. Grâce aux très faibles dimensions de l'élément suspendu 2, celui-ci présente une très faible inertie thermique et donc une réponse thermique très rapide, ce qui permet l'application d'un signal d'excitation modulé.

Le fonctionnement du dispositif de mesure de la figure 1 va maintenant être expliqué.

Le nanofil 2 suspendu est disposé dans un mélange gazeux à analyser.

Le nanofil 2 est échauffé par passage du courant alternatif. Par effet Joule, celui-ci génère une puissance thermique provoquant son auto-échauffement. L'élévation de température du nanofil dépend des transferts de chaleur du nanofil vers l'environnement gazeux et vers les ancrages. Ce sont principalement les transferts avec l'environnement qui contrôlent l'échauffement du nanofil.

Sur la figure 2, on peut voir le modèle thermique du dispositif de mesure.

Le nanofil chauffé peut s'assimiler à une capacité thermique $C_{th}$ connectée à un thermostat à température $T_0$ par le biais d'une résistance thermique $R_{th}$ qui s'assimile à la résistance du nanofil $R_{th\ nanofil}$ et la résistance de l'environnement gazeux $R_{th\ gaz}$.

Le principe de la mesure est le suivant :

Lorsque la concentration en analyte dans l'environnement gazeux est modifiée, sa conductivité thermique est modifiée. Il en résulte que les échanges thermiques entre le nanofil et l'environnement gazeux sont modifiés, c'est alors la température du système qui varie.

Afin de déterminer cette température, on détermine la résistance électrique du nanofil qui dépend de la température. La résistance thermique est déterminée en mesurant la tension aux bornes du nanofil 2 par le voltmètre.

En déterminant la température locale du nanofil, il est possible de remonter à la ou aux concentrations en analyte dans le gaz porteur.

Ce nanofil qui assure son échauffement et sert à la détection est qualifié de nanofil ou élément de premier type.

La puissance par effet Joule produite par le nanofil est égale à :

$$P_J = R_0 I^2$$

$R_0$ est la résistance électrique initiale du nanofil.

La source de courant 6 polarise le nanofil avec un courant alternatif $I = I_0 \cos(\omega t)$ de pulsation $\omega$, il en résulte une création de puissance à la pulsation $2\omega$ :

$$P_J = \frac{R_0 I_0^2}{2}[1 + \cos(2\omega t)]$$

.

Grâce à l'invention, puisque l'élément chauffant est formé par un nanofil, il présente une constante de temps thermique faible, devant l'inverse de la fréquence d'excitation thermique, il en résulte alors une variation de température dans le nanofil à la même pulsation :

$$\Delta T = \Delta T_0(1 + \cos(2\omega t)) \quad (\mathrm{I})$$

La tension aux bornes du nanofil comporte alors une harmonique à $3\omega$ dépendante de la température.

$$V_{sinw} = RI = R_0\big(1 + TCR.\Delta T_0(1 + \cos(2\omega t))\big)I_0 \cos(\omega t)$$

$$V_{3\omega} = \frac{1}{2}R_0 I_0 TCR.\Delta T_0 \cos(3\omega t)$$

Par filtrage et détection synchrone, on peut déterminer la température du nanofil en mesurant l'amplitude de cette harmonique.

La mesure en appliquant un signal d'excitation dynamique permet de s'affranchir de manière sensible des phénomènes constants extérieurs, tels une dérive lente de la température de l'environnement, qui dans un capteur de l'état de la technique peut perturber les mesures. Par ailleurs, la détection à une fréquence différente de la fréquence d'excitation permet de réduire le signal de fond, la majeure partie du signal mesuré dépend alors des variations de température dues à l'effet Joule.

Par ailleurs, grâce à l'invention, il est possible de réduire le bruit de Flicker en appliquant un signal d'excitation à une fréquence suffisamment élevée, par exemple entre 10Hz et 1MHz et avantageusement 1KHz et 10KHz.

Dans le calcul précédent, il a été fait l'hypothèse d'une élévation de température sensiblement identique tout le long du nanofil. Or, en pratique l'élévation de température dans le nanofil présente un profil parabolique.

Pour tenir compte de ce profil parabolique, la variation de résistance sur toute la longueur du nanofil est intégrée en tenant compte du profil de température dans le nanofil. Cette hypothèse ne modifie pas le principe de détection du dispositif selon l'invention.

De manière avantageuse, une mesure hétérodyne peut être effectuée. Pour cela, un deuxième courant $I_{bias}$ à

la pulsation $2\omega$ - $\Delta\omega$ est injecté dans la nanofil en plus du signal d'excitation. Le signal de sortie s'écrit alors :

$$V_{sinw} = RI = R_0\left(1 + TCR.\Delta T_0(1 + \cos(2\omega t))\right)\left[I_0\cos(\omega t) + I_{bias}\cos\left((2\omega - \Delta\omega)t\right)\right]$$

La multiplication des signaux entraîne la création d'une composante du signal à $\Delta\omega$.

$$V_{\Delta\omega} = \frac{1}{2}R_0 I_{bias} TCR.\Delta T_0 \cos(\Delta\omega t)$$

Cette méthode permet d'une part de détecter un signal à une fréquence différente de la fréquence d'excitation et d'autre part de s'affranchir de la fréquence de coupure électrique du système en ramenant le signal de détection à une fréquence basse. On obtient alors un signal de détection non atténué.

L'application de cette méthode est particulièrement intéressante dans un fonctionnement à fréquence élevée.

L'application d'un signal de bias a pour effet de provoquer un échauffement non désiré, mais celui-ci étant à une fréquence différente, il ne gêne pas les mesures.

Sur la figure 3, on peut voir un autre exemple de dispositif de mesure de flux thermique mettant en œuvre deux nanofils suspendus 2.1, 2.2 et disposés sensiblement parallèlement l'un à côté de l'autre.

Les deux nanofils 2.1, 2.2 sont suspendus à un support 4.

Les points d'ancrage de chaque nanofil 2.1, 2.2 respectivement sont désignés A et C, B et D respectivement.

La distance séparant les deux nanofils est de préférence inférieure à 10 $\mu$m et supérieure à 50 nm.

Dans le cas d'un capteur Pirani, la valeur de la distance entre les nanofils influe sur la gamme de pression dans laquelle le capteur est sensible. Le nanofil 2.1 est relié à une première source de courant alternatif 6.1 et à un premier moyen de mesure de la tension 8.1 et le nanofil 2.2 est connecté à une deuxième source de courant alternatif 6.2 et à un deuxième moyen de mesure de la tension 8.2.

Le nanofil 2.1 assure l'excitation thermique du dispositif et le nanofil 2.2 assure la détection. L'environnement gazeux à analyser est situé entre les deux nanofils. Le nanofil 2.1 est un nanofil de premier type, et le nanofil 2.2 sera désigné "nanofil de deuxième type" car il sert uniquement à la détection.

La mise en œuvre d'au moins un nanofil d'excitation et d'au moins un nanofil de détection distinct du nanofil d'excitation permet de déduire la quantité de chaleur transférée d'un élément suspendu vers l'autre élément suspendu. En outre, cette mise en œuvre permet de s'affranchir de l'influence de l'ancrage des nanofils sur le support. L'effet de dissipation thermique au niveau des ancrages agit de manière égale sur les deux nanofils et on mesure une différence de températures entre les deux nanofils.

Pour la suite, l'indice "in" désigne une caractéristique physique du nanofil 2.1 et l'indice "out" désigne une caractéristique physique du nanofil 2.2.

Le principe de la mesure est le suivant :

Le système repose sur une mesure simultanée de la température dans deux nanofils placés en regard.

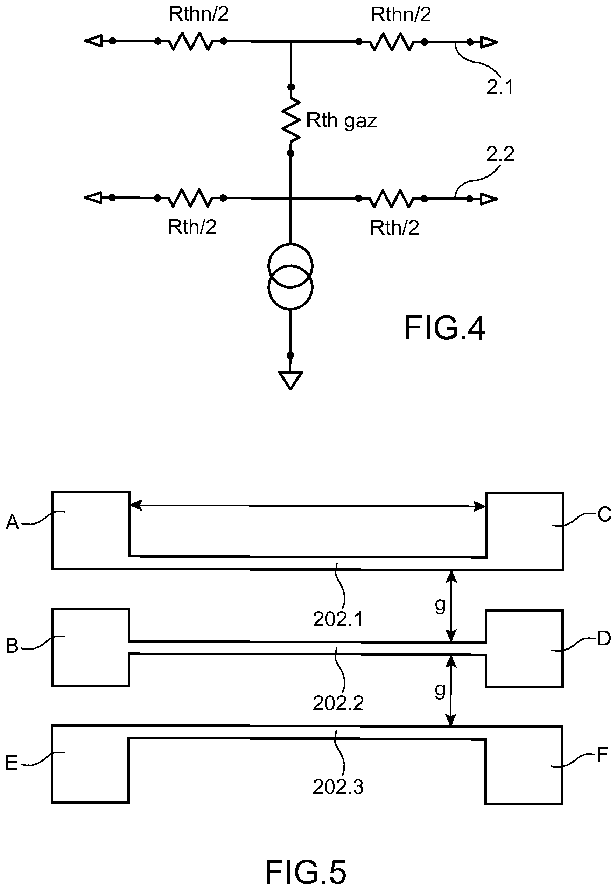

Sur la figure 4, on peut voir un schéma électrique équivalent du dispositif :

L'environnement gazeux est représenté sous la forme d'une résistance thermique reliant les deux nanofils, cette résistance est fonction :

- de la géométrie du dispositif de mesure (surface d'échange thermique, distance entre les nanofils),
- des paramètres de l'environnement gazeux, tels que la concentration, la conductivité thermique, la pression, la température....

$R_{thn}$ est la résistance thermique d'un nanofil. Dans l'exemple considéré les deux nanofils ont une résistance identique.

Le nanofil 2.1 est polarisé en courant, il génère alors une puissance thermique par effet Joule.

En reprenant le fonctionnement du capteur de la figure 1, la température du nanofil 2.1 est $\Delta T_{in} = \Delta T_{in_0}(1 + \cos(2\omega t))$.

Cette élévation dynamique de température induit une variation dynamique de la résistance électrique du nanofil 2.1 et donc de la tension à ses bornes qui s'écrit :

$$V_{in_{3\omega}} = R_{in_0} I_{in_0} TCR.\Delta T_{in_0}\cos(3\omega t) \quad (\text{II})$$

La production de chaleur dans le nanofil 2.1 entraîne un transfert de cette chaleur produite vers le nanofil 2.2 disposé en regard par l'intermédiaire de l'environnement gazeux situé entre les deux nanofils 2.1, 2.2.

Dans cette explication, on ne tient compte que de la conduction thermique, sont négligés les phénomènes de convection et d'échanges radiatifs.

La valeur de la résistance thermique du gaz peut s'écrire :

$$R_{th_{gaz}} = \frac{1}{k_{gaz}}\gamma$$

$\gamma$ est un terme en $m^{-1}$ dépendant notamment des dimensions de la « résistance » de gaz et de la pression du milieu.

Puisque la température dans le nanofil 2.1 varie à la pulsation 2w, la température du nanofil 2.2 varie à la pulsation 2w.

La variation de température dans le nanofil 2.2 s'écrit :

$$\Delta T_{out} = \Delta T_{in} \frac{R_{th_n}}{R_{th_n} + R_{th_{gaz}}} \quad (III)$$

Avec $R_{th_n}$ la résistance thermique due aux nanofils.

Le matériau et les dimensions des nanofils sont choisis de sorte que $R_{th_n} \ll R_{th_{gaz}}$, la variation de température dans le nanofil 2.2 peut s'écrire à partir de la relation (III) :

$$\Delta T_{out} \cong \Delta T_{in} \frac{R_{th_n}}{R_{th_{gaz}}} \quad (IV)$$

La variation de température dans le nanofil 2.2 est par ailleurs proportionnelle à la variation de la conductivité thermique du gaz.

Le nanofil 2.2 présente une variation de résistance électrique à la pulsation 2ω qui dépend de la température; elle s'écrit :

$$\Delta R_{out} = TCR.R_{out}\Delta T_{out_0}[1 + \cos(2\omega t)] \quad (V)$$

Le nanofil 2.2 est également polarisé pour permettre la mesure de l'amplitude de la variation de tension électrique à ses bornes. De manière avantageuse, le courant de polarisation du nanofil 2.2 est un courant constant. D'une part, cela permet d'éviter tout phénomène de croisement (ou "crosstalking") entre le signal d'excitation et celui de détection, les signaux générés variant à des fréquences différentes (3f pour le signal du nanofil in, 2f pour le signal du nanofil out), cela permet de simplifier la détection. La variation de tension aux bornes du nanofil 2.2 présente également une pulsation 2ω et s'écrit, c'est cette variation à la pulsation 2ω qui renseigne sur la température.

$$V_{out} = (R_{out} + \Delta R_{out})I_{out_0} \quad (VI)$$

La partie de la variation de tension aux bornes du nanofil 2.2 à la pulsation 2ω ($\Delta R_{out})I_{out_0}$ est égale à: en reprenant la relation (V),

$$V_{out_{2\omega}} = I_{out_0}TCR.R_{out}\Delta T_{out_0}\cos(2\omega t) \quad (VII)$$

Ainsi, à partir des relations (II) et (VII) :

$$\frac{|V_{out_{2\omega}}|}{|V_{in_{3\omega}}|} = \frac{R_{out}I_{out_0}\Delta T_{out_0}}{R_{in}I_{in_0}\Delta T_{in_0}} \quad (VIII)$$

Et comme

$$\Delta T_{out} \cong \Delta T_{in} \frac{R_{th_n}}{R_{th_{gaz}}} \quad \text{(IV)},$$

en posant

$$K = \frac{R_{out}I_{out_0}}{R_{in}I_{in_0}},$$

on obtient à partir de la relation (VIII), on peut écrire :

$$\frac{|V_{out_{2\omega}}|}{|V_{in_{3\omega}}|} = K\frac{R_{th_n}}{R_{th_{gaz}}}$$

$$R_{th_{gaz}} = KR_{th_n}\frac{|V_{in_{3\omega}}|}{|V_{out_{2\omega}}|}$$

Ce mode de mesure de la conductivité permet de supprimer l'influence des dérives sur la résistivité du matériau qui seraient dues à l'environnement. Ces variations se compensant lorsque l'on mesure le rapport

$$\frac{|V_{out_{2\omega}}|}{|V_{in_{3\omega}}|} = K\frac{R_{th_n}}{R_{th_{gaz}}}$$

En mesurant les tensions aux bornes des nanofils 2.1 et 2.2 on peut en déduire la variation de la résistance thermique du gaz entre les deux nanofils, déterminer la conductivité thermique de l'environnement gazeux et ainsi en déduire sa concentration ou sa pression. Cette détermination est indépendante des variations de l'environnement extérieur, telles que les variations de température.

Comme pour le dispositif de mesure à un nanofil, une mesure hétérodyne peut être avantageusement effectuée en injectant dans le nanofil de détection 2.2, un courant de pulsation $2\omega$ - $\Delta\omega$. Ainsi le signal électrique généré dans le nanofil de détection 2.2 présente une composante à $\Delta\omega$ dépendante de la température.

Le capteur de flux thermique à un ou plusieurs nanofils en fonctionnement dynamique peut être utilisé pour réaliser un capteur TCD pour la mesure de concentrations gazeuses ou pour réaliser une jauge Pirani pour la mesure d'une pression. La gamme de pression dans laquelle le capteur est performant dépend de la valeur de la distance séparant les nanofils. Par exemple, pour une distance de 120 nm le capteur est sensible dans une gamme allant d'environ 1 mBar à environ 100 Bar. Dans le cas d'une jauge Pirani, le choix de la valeur de la distance g séparant les deux nanofils 2.1, 2.2 permet de déterminer la gamme de pression accessibles au capteur.

Sur la figure 5, on peut voir un autre exemple de réalisation d'un dispositif de mesure selon la présente invention comportant trois nanofils 201.1, 201.2, 201.3 suspendus les uns à côtés des autres sur un support, sensiblement parallèlement. Chaque nanofil est distant du nanofil directement voisin d'une distance g. En variante, on peut envisager que les nanofils ne soient pas espacés de la même distance.

Les points d'ancrage de chaque nanofil 202.1, 202.2, 202.3 sont désignés A et C, B et D, E et F respectivement.

Les nanofils peuvent avoir une fonction d'excitation ou une fonction de détection.

Dans un exemple, le nanofil 202.2 situé entre les nanofils 202.1 et 202.3 est le nanofil d'excitation et les nanofils 202.1 et 202.3 forment les nanofils de détection. Il est alors possible de réaliser une mesure différentielle en appliquant le signal d'excitation sur le nanofil central 202.2 et combinant les mesures obtenues avec chacun des nanofils 202.1 et 202.3.

A des fins de simplicité, les moyens de polarisation et les moyens de détection connectés à chacun des nanofils ne sont pas représentés.

Dans un autre exemple, les nanofils 202.1 et 202.3 forment des nanofils d'excitation et le nanofil 202.2 forme un nanofil de détection. Ce dispositif permet d'augmenter l'amplitude des variations de température dans le nanofil central 202.2.

Dans un autre exemple, l'un des deux nanofils d'extrémité 202.1 ou 202.3 est utilisé comme nanofil d'excitation et l'autre nanofil d'extrémité 202.3 ou 202.1 et le nanofil central sont utilisés comme nanofils de détection.

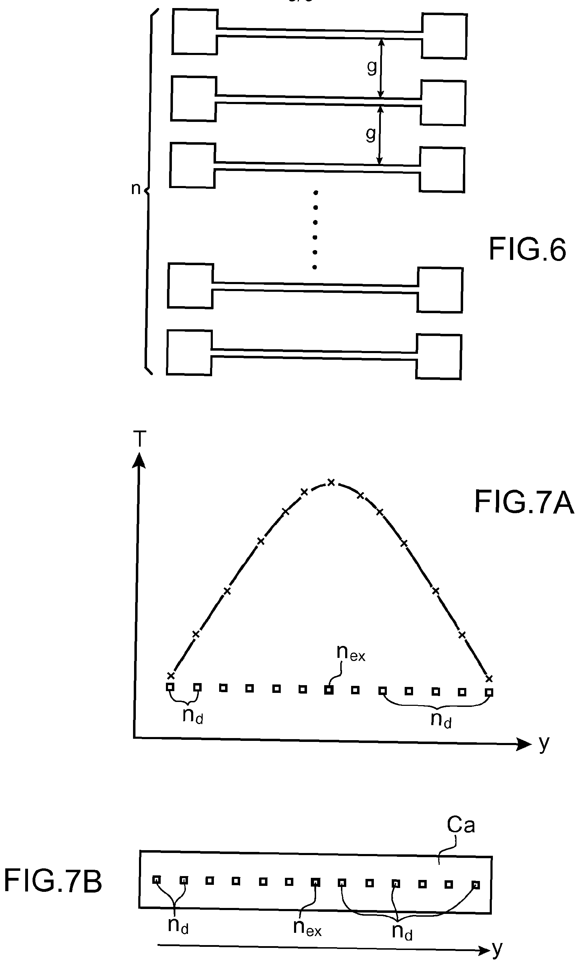

Sur la figure 6, on peut voir un autre exemple de réalisation dans lequel le dispositif comporte n nanofils disposés parallèlement les uns par rapport aux autres et distant deux à deux d'une distance g, n étant un entier positif strictement supérieur à 1, les cas n = 1, n = 2 et n = 3 ont déjà été décrits ci-dessus en relation avec les figures 1 à 5.

De manière avantageuse, le capteur est destiné à être disposé dans un canal de sorte que les n nanofils soient disposés dans la largeur du canal fluidique.

Dans un exemple avantageux, le dispositif comporte n nanofils simples placés les uns à côtés des autres dans un plan en alternant un nanofil d'excitation et un nanofil de détection. Les nanofils d'excitation sont connectés en parallèle et les nanofils de détection sont connectés en parallèle, on obtient ainsi un capteur dont la résolution est augmentée.

On effectue alors un moyennage spatial du bruit sur un grand nombre d'échantillons réduisant de fait le bruit d'un facteur $\sqrt{n}$. Une telle réalisation permet également d'augmenter la surface d'échange en regard entre les nanofils d'excitation et les nanofils de détection.

Du point de vue du bruit et en considérant toujours le même courant circulant dans chacun des nanofils, la mise en parallèle de n résistances permet de réduire les bruits de Johnson et de Flicker en diminuant la valeur de la résistance tout en gardant le même signal utile.

La connexion en parallèle peut être obtenue par des connexions croisées, réalisées avec au moins deux niveaux de métallisation.

En outre de manière très avantageuse, un tel dispositif de mesure peut permettre de caractériser les échanges thermiques dans un milieu, par exemple de déterminer le profil de température dans la largeur du canal.

Sur la figure 7A, on peut voir représenter de manière schématique la température T de chacun des n nanofils en fonction de sa position transversale y dans le canal Ca représenté sur la figure 7B. Le nanofil désigné $n_{ex}$ situé au centre forme le nanofil d'excitation et les nanofils désignés $n_d$ forment des nanofils de détection. On obtient alors le profil de température transversal de l'intérieur du canal Ca.

Cette mesure permet de caractériser des transferts de chaleur dans le canal fluidique puisqu'elle donne une information sur les échanges de chaleur par le gaz.

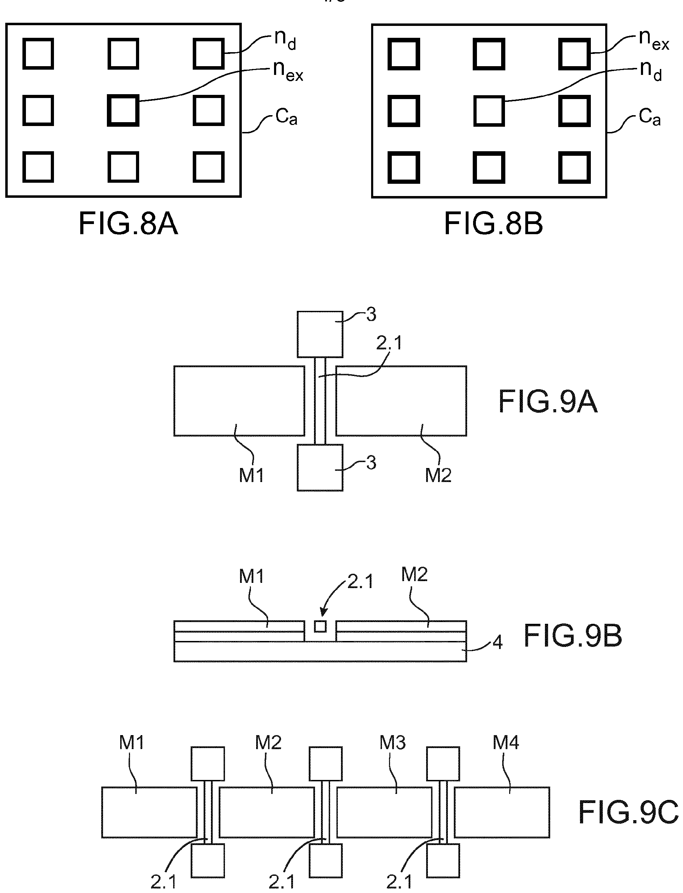

Sur les figures 8A et 8B, on peut voir un autre exemple de réalisation d'un dispositif de mesure dans lequel les nanofils sont répartis en couches parallèles et sont donc distribués dans plusieurs plans. Le capteur s'étend alors dans trois dimensions.

Les nanofils se distribuent suivant la largeur du canal et sa hauteur. Le canal Ca en coupe transversale est représenté. Dans cet exemple, le canal a une section rectangulaire.

Par exemple, sur la figure 8A le nanofil d'excitation est le nanofil central désigné $n_{ex}$, les autres nanofils $n_d$ étant des nanofils de détection. Cette configuration permet de caractériser les échanges thermiques suivant la largeur et la hauteur du canal.

Sur la figure 8B, les nanofils périphériques $n_{ex}$ forment les nanofils d'excitation et le nanofil central $n_d$ forme le nanofil de détection. On augmente alors l'amplitude des variations de température dans le nanofil central $n_d$.

Il sera compris que les dispositions des figures 8A à 8B sont présentées à titre d'exemples non limitatifs et que d'autres dispositions et/ou nombre de nanofils d'excitation et de détection entrent dans le cadre de la présente invention. Par exemple, les nanofils peuvent être distribués en couronne autour d'un nanofil central, ainsi tous les nanofils de la couronne sont à équidistance du nanofil central.

Sur les figures 9A à 9D, on peut voir des exemples de réalisation d'un dispositif de mesure de flux thermique selon la présente invention comportant un ou plusieurs des éléments suspendus du premier type, i.e. formant à la fois des éléments d'excitation et de détection et des masses thermostatées.

Sur la figure 9A, on peut voir un nanofil 2.1 entouré de masses thermostatées M1 et M2 situées de part et d'autre du fil. Les masses thermostatées sont destinées à améliorer l'évacuation de la chaleur produite à la nanofil 2.1. Par exemple ces masses sont à la température du support du dispositif de mesure.

Sur la figure 9B, on peut voir une vue en coupe du dispositif de la figure 9A, dans cet exemple les masses thermostatées sont formées par des motifs de silicium à la température du support, ces motifs étant réalisés en même temps que le nanofil 2.1.

Sur la figure 9C, on peut voir un exemple de dispositif comportant n éléments suspendus du premier type uniquement 2.1, et n + 1 masses thermostatées M1 à $M_{n+1}$ s'intercalant entre les nanofils de sorte que chaque nanofil soit encadré par deux masses. Dans l'exemple représenté, n est égal à 3.

Chaque nanofil est relié à une source de courant alternatif de même fréquence ou de fréquence différente dont l'intensité assure un échauffement du nanofil correspondant par effet Joule.

La mise en œuvre des masses thermostatées à la température du support permet d'augmenter la surface d'échange entre le thermostat formé par le support et les masses thermostatées et le nanofil et donc d'augmenter les échanges avec le gaz ce qui a pour effet d'améliorer la sensibilité du dispositif de mesure.

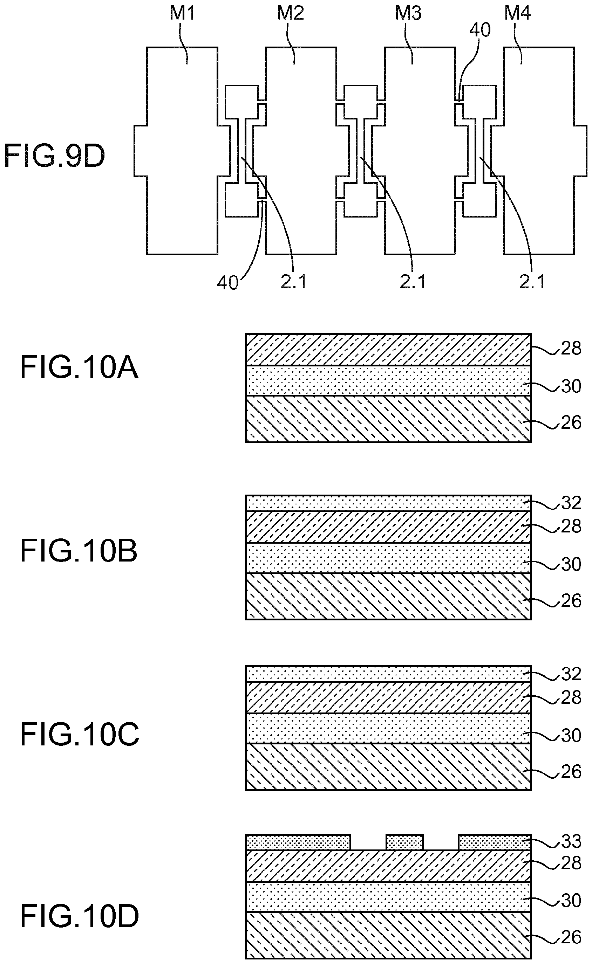

Sur la figure 9D, le dispositif comporte n éléments suspendus du premier type 2.1 et n+1 masses thermostatées M1 à $M_{n+1}$ à la température du support. Les n éléments du premier type sont connectés en parallèle par des connexions 40 formées dans un deuxième niveau de métallisation. Dans l'exemple représenté, n est égal à 3. Il sera compris que n peut être quelconque.

Nous allons maintenant décrire un exemple de procédé de fabrication d'un capteur selon la présente invention.

Sur les figures 10A à 10H, on peut voir des représentations schématiques de différentes étapes du procédé de réalisation.

Dans l'exemple décrit, on utilise une plaque de SOI (Silicon On Insulator) en terminologie anglaise ou silicium sur isolant, représenté sur la figure 10A. Le substrat SOI comporte une couche de silicium 26, une couche de silicium monocristallin 28, les couches 26, 28 étant séparées par une couche de $SiO_2$ 30. La couche de silicium 28 monocristallin forme la face avant.

Lors d'une première étape une couche de d'oxyde $SiO_2$ 32 est déposée sur la couche 28. L'élément ainsi formé est représenté sur la figure 10B.

Lors d'une étape suivante, on effectue un dopage P++ par exemple au bore, de la couche de silicium 28 située entre la couche d'oxyde 30 et la couche d'oxyde 32.

Le dopage au travers de la couche d'oxyde permet une répartition plus homogène des dopants dans la couche 28. Le dopage obtenu est de l'ordre de $1.10^{19}$ at./cm$^3$). Ce dopage a pour effet de maximiser le coefficient de température de résistance du silicium.

L'élément ainsi formé est représenté sur la figure 10C. Le dopage est symbolisé par des points.

Lors d'une étape suivante, on retire la couche d'oxyde 32 et on dépose une couche de résine 33, dans laquelle définit les contours des motifs dans la résine 32 par lithographie, par exemple par lithographie en UV profond (DUV pour Deep-UV en en terminologie anglaise) ou par une lithographie hybride DUV et à faisceau d'électrons (e-beam en terminologie anglaise). Ces procédé de lithographie sont bien connus de l'homme du métier et ne seront pas décrits en détail. La lithographie e-beam permet de s'affranchir des effets liés à la diffraction de la lumière lors de la gravure de dispositifs nanométriques.

L'élément ainsi formé est représenté sur la figure 10D.

Lors d'une étape suivante, la couche de silicium est gravée, par exemple par gravure ionique réactive ou RIE (Reactive Ion Etching en anglais) anisotrope.

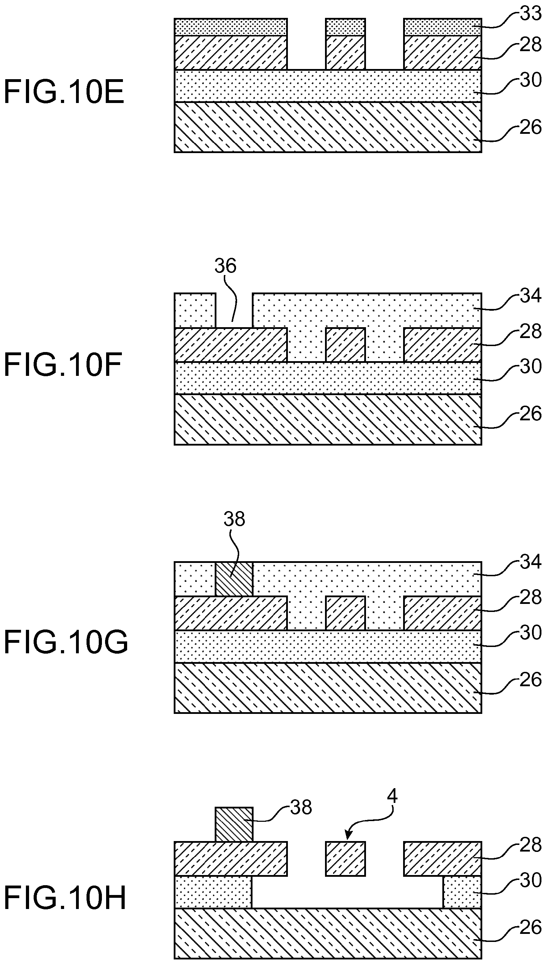

L'élément ainsi formé est représenté sur la figure 10E.

Lors d'une étape suivante, on effectue un dépôt chimique de $SiO_2$ 34 sur la couche de silicium gravée 28 qui est ensuite gravé pour délimiter les emplacements 36 des contacts électriques, par exemple gravure plasma.

L'élément ainsi obtenu est représenté sur la figure 10F.

On réalise ensuite les contacts électriques 38 en déposant par exemple de l'aluminium, par exemple dépôt par pulvérisation.

L'élément ainsi obtenu est représenté sur la figure 10G.

Lors d'une étape suivante, le nanofil est libéré, par exemple en gravant la couche 30, par exemple avec de l'acide fluorhydrique vapeur.

La structure libérée est visible sur la figure 10H.

Le capteur de flux thermique selon l'invention permet de réaliser un capteur de concentration de gaz ou un capteur Pirani qui est indépendant de la variation des paramètres de l'environnement extérieur tel que les variations de température.

Ce capteur est particulièrement adapté pour être associé à une microcolonne de chromatographie en phase gazeuse. Un ou plusieurs capteurs sont disposés dans un canal connectée en série en sortie de la microcolonne et permet de détecter les pics d'analytes.

**Revendications**

1. Capteur de flux thermique comportant au moins deux éléments suspendus par rapport à un support, lesdits au moins deux éléments suspendus étant formés par n premiers éléments suspendus (2, 2.1, 202.1) par rapport au support, n étant un entier positif supérieur ou égal à 2, chaque premier élément suspendu (2, 2.1, 202.1) étant en un matériau conducteur électrique, des premiers moyens de polarisation (6, 6.1) de chaque premier élément suspendu (2, 2.1, 202.1) et des premiers moyens de mesure (8, 8.1) de l'amplitude de variation de la tension électrique aux bornes de chaque premier élément suspendu (2, 2.1, 202.1), chaque premier élément suspendu (2, 2.1, 202.1) étant formé par au moins un nanofil et lesdits premiers moyens de polarisation (6, 6.1) sont formés par une source de courant alternatif dont l'intensité assure un échauffement de chaque premier élément suspendu (2, 2.1, 202.1) par effet Joule.

**2.** Capteur de flux thermique selon la revendication 1, dans lequel les n premiers éléments suspendus (202.3) sont disposés sensiblement parallèlement entre eux.

**3.** Capteur de flux thermique selon la revendication 1 ou 2, comportant au moins deux masses thermiques reliées au support, les masses thermiques étant disposées par de part et d'autre du premier élément suspendu, avantageusement ledit capteur comportant n premiers éléments suspendus et n+1 masses thermiques, n étant supérieur ou égal à 2, lesdites n + 1 masses thermiques étant disposées de sorte que chaque premier élément est encadré par deux masses.

**4.** Capteur de flux thermique comportant au moins deux éléments suspendus par rapport à un support, lesdits au moins deux éléments suspendus étant formés par n premiers éléments suspendus (2, 2.1, 202.1) par rapport au support, n étant un entier positif supérieur ou égal à 1, et m deuxièmes éléments comportant m deuxième éléments suspendus, m étant un entier positif supérieur ou égal à 1, chaque premier élément suspendu (2, 2.1, 202.1) étant en un matériau conducteur électrique, des premiers moyens de polarisation (6, 6.1) de chaque premier élément suspendu (2, 2.1, 202.1) et des premiers moyens de mesure (8, 8.1) de l'amplitude de variation de la tension électrique aux bornes de chaque premier élément suspendu (2, 2.1, 202.1), chaque premier élément suspendu (2, 2.1, 202.1) étant formé par au moins un nanofil et lesdits premiers moyens de polarisation (6, 6.1) sont formés par une source de courant alternatif dont l'intensité assure un échauffement de chaque premier élément suspendu (2, 2.1, 202.1) par effet Joule, chaque deuxième élément suspendu comportant des deuxièmes moyens de polarisation (6.2) de chaque deuxième élément suspendu (2.2, 202.2) et des deuxièmes moyens de mesure (8.2) de l'amplitude de la variation de la tension électrique aux bornes de chaque deuxième élément suspendu (2.2, 202.2), les deuxièmes moyens de polarisation (6.2) étant formés par une source de courant alternatif de fréquence différente de celle des premiers moyens de polarisation ou par une source de courant constant.

**5.** Capteur de flux thermique selon la revendication 4, dans lequel chaque deuxième élément est formé par au moins un nanofil, sensiblement parallèle au premier élément suspendu (2.2, 202.2) et distant du premier élément suspendu (2.2, 202.2) d'une distance donnée g, et dans lequel les deuxièmes moyens de mesure (8.2) de l'amplitude de la variation de la tension électrique aux bornes de chaque deuxième élément suspendu (2.2, 202.2) permettent de déduire la quantité de chaleur transférée d'un premier élément suspendu vers un deuxième élément suspendu.

**6.** Capteur de flux thermique selon la revendication 4 ou 5, dans lequel les m deuxièmes éléments suspendus sont sensiblement parallèles entre eux.

**7.** Capteur de flux thermique selon l'une des revendications 4 à 6, dans lequel le premier élément suspendu est encadré entre m deuxièmes éléments suspendus et/ou le deuxième élément suspendu est encadré entre n premiers éléments suspendus.

**8.** Capteur de flux thermique selon les revendications 4 à 7, dans lequel les n premiers éléments suspendus et les m deuxièmes éléments suspendus sont répartis dans un plan ou plusieurs plans parallèles.

**9.** Capteur de flux thermique selon l'une des revendications 4 à 8, dans lequel ledit capteur de flux thermique comporte un premier élément suspendu encadré par m deuxièmes éléments suspendus, est destiné à être disposé dans un canal (Ca), lesdits éléments suspendus étant disposés parallèlement à l'axe du canal (Ca) de sorte à déterminer les échanges de chaleur transversaux dans le gaz à l'intérieur du canal (Ca).

**10.** Capteur de flux thermique selon l'une des revendications 1 à 9, dans lequel les premiers moyens de polarisation des n premiers éléments suspendus sont communs et/ou les deuxièmes moyens de polarisation des m seconds éléments suspendus (6.2) sont communs.

**11.** Capteur de flux thermique selon l'une des revendications 1 à 10, dans lequel le ou les premiers ou seconds éléments suspendus (2) ont respectivement une largeur comprise entre 10 nm et $1\mu$m, une longueur comprise entre $1\mu$m et $100\mu$m, une épaisseur comprise entre 10 nm et $1\mu$m.

**12.** Capteur de flux thermique selon la revendication 5, dans lequel la distance g séparant deux éléments suspendus est comprise entre 50 nm et 10 $\mu$m.

**13.** Système de détermination de la concentration d'un environnement gazeux comportant au moins un capteur de flux thermique selon l'une des revendications 1 à 12, une électronique de traitement des valeurs de tension électrique délivrées par le capteur de flux thermique.

14. Système de détermination de la pression dans un environnement gazeux comportant au moins un capteur de flux thermique selon l'une des revendications 1 à 12, une électronique de traitement des valeurs de tension électrique délivrées par le capteur de flux thermique.

15. Dispositif d'analyse d'un gaz ou mélange de gaz comportant une colonne de chromatographie en phase gazeuse et au moins un système de détermination de la concentration selon la revendication 13, ledit système de détermination étant disposé dans un canal connecté à la sortie de la colonne de chromatographie en phase gazeuse.


**Patentansprüche**

1. Wärmeflusssensor, umfassend wenigstens zwei Elemente, die mit Bezug zu einem Träger aufgehängt sind, wobei die wenigstens zwei aufgehängten Elemente durch n erste Elemente (2, 2.1, 202.1) gebildet sind, die mit Bezug zu dem Träger aufgehängt sind,
wobei n eine positive ganze Zahl größer oder gleich 2 ist, wobei jedes erste aufgehängte Element (2, 2.1, 202.1) aus einem elektrisch leitenden Material ist, sowie erste Polarisationsmittel (6, 6.1) für jedes erste aufgehängte Element (2, 2.1, 202.1) und erste Messmittel (8, 8.1) für die Amplitude der Variation der elektrischen Spannung an den Anschlüssen jedes ersten aufgehängten Elements (2, 2.1, 202.1), wobei jedes erste aufgehängte Element (2, 2.1, 202.1) durch wenigstens einen Nanodraht gebildet ist, und die ersten Polarisationsmittel (6, 6.1) durch eine Wechselstromquelle gebildet sind, deren Intensität eine Aufheizung jedes ersten aufgehängten Elements (2, 2.1, 202.1) mittels Joule-Effekt gewährleistet.

2. Wärmeflusssensor nach Anspruch 1, bei dem die n ersten aufgehängten Elemente (202.3) im Wesentlichen parallel zueinander angeordnet sind.

3. Wärmeflusssensor nach Anspruch 1 oder 2, umfassend wenigstens zwei thermische Massen, die mit dem Träger verbunden sind, wobei die thermischen Massen auf beiden Seiten des ersten aufgehängten Elements angeordnet sind, wobei der Sensor vorzugsweise n erste aufgehängte Elemente und n + 1 thermische Massen umfasst, wobei n größer oder gleich 2 ist, wobei die n + 1 thermischen Massen derart angeordnet sind, dass jedes erste Element durch zwei Massen eingerahmt ist.

4. Wärmeflusssensor, umfassend wenigstens zwei Elemente, die mit Bezug zu einem Träger aufgehängt sind, wobei die wenigstens zwei aufgehängten Elemente durch n erste Elemente (2, 2.1, 202.1) gebildet sind, die mit Bezug zu dem Träger aufgehängt sind, wobei n eine positive ganze Zahl größer oder gleich 1 ist, und m zweite Elemente, umfassend m zweite aufgehängte Elemente, wobei m eine positive ganze Zahl größer oder gleich 1 ist, wobei jedes erste aufgehängte Element (2, 2.1, 202.1) aus einem elektrisch leitenden Material ist, sowie erste Polarisationsmittel (6, 6.1) für jedes erste aufgehängte Element (2, 2.1, 202.1) und erste Messmittel (8, 8.1) für die Amplitude der Variation der elektrischen Spannung an den Anschlüssen jedes ersten aufgehängten Elements (2, 2.1, 202.1), wobei jedes erste aufgehängte Element (2, 201, 202.1) durch wenigstens einen Nanodraht gebildet ist, und wobei die ersten Polarisationsmittel (6, 6.1) durch eine Wechselstromquelle gebildet sind, deren Intensität eine Aufheizung jedes ersten aufgehängten Elements (2, 2.1, 202.1) mittels Joule-Effekt gewährleistet, wobei jedes zweite aufgehängte Element zweite Polarisationsmittel (6.2) für jedes zweite aufgehängte Element (2.2, 202.2) und zweite Messmittel (8.2) für die Amplitude der Variation der elektrischen Spannung an den Anschlüssen jedes zweiten aufgehängten Elements (2.2, 202.2) umfasst, wobei die zweiten Polarisationsmittel (6.2) durch eine Wechselstromquelle mit einer Frequenz gebildet sind, die verschieden ist von jener der ersten Polarisationsmittel, oder durch eine Gleichstromquelle.

5. Wärmeflusssensor nach Anspruch 4, bei dem jedes zweite Element durch wenigstens einen Nanodraht gebildet ist, der im Wesentlichen parallel zu dem ersten aufgehängten Element (2.2, 202.2) und um einen gegebenen Abstand g von dem ersten aufgehängten Element (2.2, 202.2) beabstandet ist, und bei dem die zweiten Messmittel (8.2) für die Amplitude der Variation der elektrischen Spannung an den Anschlüssen jedes zweiten aufgehängten Elements (2.2, 202.2) die Ermittlung der Menge der Wärme erlauben, die von einem ersten aufgehängten Element in Richtung eines zweiten aufgehängten Elements transferiert wird.

6. Wärmeflusssensor nach Anspruch 4 oder 5, bei dem die m zweiten aufgehängten Elemente im Wesentlichen zueinander parallel sind.

**7.** Wärmeflusssensor nach einem der Ansprüche 4 bis 6, bei dem das erste aufgehängte Element zwischen m zweiten aufgehängten Elementen eingerahmt ist, und/oder das zweite aufgehängte Element zwischen n ersten aufgehängten Elementen eingerahmt ist.

**8.** Wärmeflusssensor nach den Ansprüchen 4 bis 7, bei dem die n ersten aufgehängten Elemente und die m zweiten aufgehängten Elemente in einer Ebene oder mehreren parallelen Ebenen verteilt sind.

**9.** Wärmeflusssensor nach einem der Ansprüche 4 bis 8, bei dem der Wärmeflusssensor ein erstes aufgehängtes Element umfasst, das durch m zweite aufgehängte Elemente eingerahmt ist, und dazu ausgelegt ist, in einem Kanal (Ca) angeordnet zu sein, wobei die aufgehängten Elemente parallel zur Achse des Kanals (Ca) derart angeordnet sind, dass die transversalen Wärmeaustauschvorgänge in dem Gas im Inneren des Kanals (Ca) bestimmt werden.

**10.** Wärmeflusssensor nach einem der Ansprüche 1 bis 9, bei dem die ersten Polarisationsmittel der n ersten aufgehängten Elemente gemeinsam sind und/oder die zweiten Polarisationsmittel der m zweiten aufgehängten Elemente (6.2) gemeinsam sind.

**11.** Wärmeflusssensor nach einem der Ansprüche 1 bis 10, bei dem das oder die erste oder zweite aufgehängte Elemente (2) jeweils eine Breite haben, die zwischen 10 nm und 1 $\mu$m enthalten ist, eine Länge, die zwischen 1 $\mu$m und 100 $\mu$m enthalten ist, eine Dicke, die zwischen 10 nm und 1 $\mu$m enthalten ist.

**12.** Wärmeflusssensor nach Anspruch 5, bei dem der Abstand g, der zwei aufgehängte Elemente separiert, zwischen 50 nm und 10 $\mu$m enthalten ist.

**13.** System zur Bestimmung der Konzentration einer Gasumgebung, umfassend wenigstens einen Wärmeflusssensor nach einem der Ansprüche 1 bis 12, sowie eine Elektronik zur Verarbeitung der Werte der elektrischen Spannung, die durch den Wärmeflusssensor geliefert werden.

**14.** System zur Bestimmung des Drucks in einer Gasumgebung, umfassend wenigstens einen Wärmeflusssensor nach einem der Ansprüche 1 bis 12, sowie eine Elektronik zur Verarbeitung der Werte der elektrischen Spannung, die durch den Wärmeflusssensor geliefert werden.

**15.** Vorrichtung zur Analyse eines Gases oder einer Gasmischung, umfassend eine Gasphasenchromatographiesäule und wenigstens ein System zur Bestimmung der Konzentration nach Anspruch 13, wobei das System zur Bestimmung in einem Kanal angeordnet ist, der an den Ausgang der Gasphasenchromatographiesäule angeschlossen ist.

**Claims**

**1.** A thermal flow sensor comprising at least two elements suspended with respect to a support, said at least two suspended elements being formed by n first suspended elements (2, 2.1, 202.1) with respect to a support, n being a positive integer higher than or equal to 2, , each first suspended element (2, 2.1, 202.1) being of an electrically conductive material, first means (6, 6.1) for biasing each first suspended element (2, 2.1, 202.1) and first means (8, 8.1) for measuring the variation amplitude of the electric voltage at the terminals of each first suspended element (2, 2.1, 202.1), each first suspended element (2, 2.1, 202.1) being formed by at least one nanowire and said first biasing means (6, 6.1) being formed by an alternating current source the intensity of which provides heating of each first suspended element (2, 2.1, 202.1) by Joule effect.

**2.** The thermal flow sensor according to claim 1, wherein the n first suspended elements (202.3) are provided substantially parallel to each other.

**3.** The thermal flow sensor according to claim 1 or 2, comprising at least two thermal masses linked to the support, the thermal masses being provided on either side of the first suspended element, advantageously the sensor comprising n first suspended elements and n + 1 thermal masses, n being higher than or equal to 2, said n + 1 thermal masses being disposed such that each first element is surrounded by two masses.

**4.** A thermal flow sensor comprising at least two elements suspended with respect to a support, said at least two suspended elements being formed by n first suspended elements (2, 2.1, 202.1) with respect to a support, n being a positive integer higher than or equal to 1, and m second elements comprising m second suspended elements, m being a positive integer higher than or equal to 1, each first suspended element (2, 2.1, 202.1) being of an electrically conductive material, first means (6, 6.1) for biasing each first suspended element (2, 2.1, 202.1) and first means (8, 8.1) for measuring the variation amplitude of the electric voltage at the terminals of each first suspended element (2, 2.1, 202.1), each first suspended element (2, 2.1, 202.1) being formed by at least one nanowire and said first biasing means (6, 6.1) being formed by an alternating current source the intensity of which provides heating of each first suspended element (2, 2.1, 202.1) by Joule effect, each second suspended element comprising second means (6.2) for biasing each second suspended element (2.2, 202.2) and second means (8.2) for measuring the variation amplitude of the electric voltage at the terminals of each second suspended element (2.2, 202.2), the second biasing means (6.2) being formed by an alternating current source of a frequency different from that of the first biasing means or by a direct current source.

5. The thermal flow sensor according to claim 4, wherein each second element is formed by at least one nanowire, substantially parallel to the first suspended element (2.2, 202.2) and away from the first suspended element (2.2, 202.2) by a given distance (g), and wherein the second means (8.2) for measuring the variation amplitude of the electric voltage at the terminals of each second suspended element (2.2, 202.2) enable the quantity of heat transferred from a first suspended element to a second suspended element to be deduced.

6. The thermal flow sensor according to claim 4 or 5, wherein the m second suspended elements are substantially parallel with each other.

7. The thermal flow sensor according to one of the claims 4 to 6, wherein the first suspended element is surrounded with m second suspended elements and/or the second suspended element is surrounded between n first suspended elements.

8. The thermal flow sensor according to claims 4 to 7, wherein the n first suspended elements and m second suspended elements are distributed in a plane or several parallel planes.

9. The thermal flow sensor according to one of claims 4 to 8, wherein said thermal flow sensor comprising a first suspended element surrounded by m second suspended elements, is intended to be disposed in a channel (Ca), said suspended elements being disposed parallel to the axis of the channel (Ca) so as to determine the transverse heat exchanges in the gas inside the channel (Ca).

10. The thermal flow sensor according to one of the claims 1 to 9, wherein the first means for biasing the n first suspended elements are common and/or the second means for biasing the m seconds suspended elements (6.2) are common.

11. The thermal flow sensor according to one of claims 1 to 10, wherein the first or second suspended elements (2) have respectively a width between 10 nm and 1 $\mu$m, a length between 1 $\mu$m and 100 $\mu$m, a thickness between 10 nm and 1 $\mu$m.

12. The thermal flow sensor according to claim 5, wherein the distance g separating two suspended elements is between 50 nm and 10 $\mu$m.

13. A system for determining the concentration of a gas environment comprising at least one thermal flow sensor according to one of claims 1 to 12, an electronics for processing the electric voltage values delivered by the thermal flow sensor.

14. A system for determining the pressure in a gas environment comprising at least one thermal flow sensor according to one of claims 1 to 12, an electronics for processing the electric voltage values delivered by the thermal flow sensor.

15. A device for analysing a gas or gas mixture comprising a gas chromatography column and at least one system for determining the concentration according to claim 13, said determining system being disposed in a channel connected to the output of the gas chromatography column.

FIG.1

FIG.2

FIG.3

Rthn/2    Rthn/2

2.1

Rth gaz

2.2

Rth/2    Rth/2

FIG.4

A    C

202.1

g

B    D

202.2

g

E    F

202.3

FIG.5

FIG.6

FIG.7A

$n_{ex}$

$n_d$

$n_d$

FIG.7B

Ca

$n_d$

$n_{ex}$

$n_d$

$n_d$

$n_{ex}$

$C_a$

## FIG.8A

$n_{ex}$

$n_d$

$C_a$

## FIG.8B

3

2.1

## FIG.9A

M1 M2

3

M1 2.1 M2

4

## FIG.9B

M1 M2 M3 M4

## FIG.9C

2.1 2.1 2.1

FIG.9D

M1    M2    M3    40    M4

40    2.1    2.1    2.1

FIG.10A

28
30
26

FIG.10B

32
28
30
26

FIG.10C

32
28
30
26

FIG.10D

33
28
30
26

FIG.10E

FIG.10F

FIG.10G

FIG.10H

## RÉFÉRENCES CITÉES DANS LA DESCRIPTION

**Documents brevets cités dans la description**

• WO 2001044547 A

**Littérature non-brevet citée dans la description**

• **WEI et al.** Single-walled carbon nanotube pirani vacuum gauge. *dans Proceedings of the 2010 10th Solid State and Integrated Circuit Technology (ICSICT) Conférence,* 01 Novembre 2010, 1407-1409